# EUROPEAN PATENT SPECIFICATION

(11) **EP 1 022 273 B1**
(45) Date of publication and mention of the grant of the patent: **14.07.2004**
(21) Application number: 99300442.3
(22) Date of filing: 21.01.1999
(51) Int. Cl.: C07D 251/70, C07D 251/52, C07D 251/46, C09D 17/00, C09D 7/02, C09D 11/02, C09B 67/00

(54) **Pigment composition containing a pigment dispersing agent**
Pigmentzubereitungen, die Pigmentdispergiermittel enthält
Compositions pigmentaires contenant un agent dispersant de pigment

(43) Date of publication of application: 26.07.2000
(73) Proprietor: TOYO INK MANUFACTURING CO., LTD., Tokyo (JP)
(72) Inventor: Kamikubo, Takashi, Chuo-ku, Tokyo (JP); Hirasawa, Yuuji, Chuo-ku, Tokyo (JP); Omura, Toru, Chuo-ku, Tokyo (JP)
(74) Representative: Woods, Geoffrey Corlett

(56) References cited:
- EP-A- 0 062 824
- EP-A- 0 074 589
- EP-A- 0 308 800
- EP-A- 0 710 706
- EP-A- 0 753 542
- DE-A- 3 222 965
- GB-A- 2 149 808
- GB-A- 2 158 837
- GB-A- 2 191 210
- GB-A- 2 266 893
- CHEMICAL ABSTRACTS, vol. 111, no. 4, 24 July 1989 Columbus, Ohio, US; abstract no. 25008, HAYASHI, MIKIO ET AL: "Dispersants for pigments" XP002107009 -& DATABASE CAPLUS Chemical Abstracts Service CA111:25008, XP002107012 & JP 63 305173 A (TOYO INK MFG. CO., LTD., JAPAN)
- CHEMICAL ABSTRACTS, vol. 109, no. 26, 26 December 1988 Columbus, Ohio, US; abstract no. 232743, HIRAKI, MASAHIRO ET AL: "Yellow heterocyclic azo dyes for paper, leather, and cotton" XP002107010 -& DATABASE CAPLUS Chemical Abstracts Service CA109:232743, XP002107013 & JP 63 099383 A (NIPPON KAYAKU CO., LTD., JAPAN)
- CHEMICAL ABSTRACTS, vol. 106, no. 22, 1 June 1987 Columbus, Ohio, US; abstract no. 178189, EHASHI, SHIGEYUKI ET AL: "Pigment dispersants" XP002107011 -& DATABASE CAPLUS Chemical Abstracts Service CA106:178189, XP002107014 & JP 61 246261 A (TOYO INK MFG. CO., LTD., JAPAN)

## Description

The present invention relates to a pigment composition comprising a pigment dispersing agent excellent in suitability in use, particularly excellent in freedom of aggregation, non-crystallizability and fluidity. The pigment composition may be an ink or a coating composition.

In a coating composition or an ink composition, a pigment which exhibits a clear color tone and a high tinting strength is generally formed of fine particles. When fine particles of a pigment are dispersed in a non-aqueous vehicle for an offset ink, a gravure ink or a coating composition, it is difficult to obtain a stable dispersion, and it is known that the dispersion is therefore stabilized by improving the affinity between the pigment and the vehicle with a pigment dispersing agent.

Many proposals for pigment dispersing agents of the above type have been so far published with regard to pigments among which copper, phthalocyanine and quinacridone pigments are mainly there.

Pigment dispersing agents having the structure of an organic dyestuff bonded to an aromatic carbon atom of a substituted triazine are know. Chemical Abstracts, vol. 106, no. 22 and European patent application EP-A-0 710 706 disclose such dispersing agents. EP-A-0 308 800 discloses pigment formulations comprising trisubstituted triazines coated on, or mixed with, the pigment.

As disclosed in Japanese Patent Publication No. 41-2466 and U.S. Patent 2855403, there are known methods of incorporating compounds obtained by introducing a side-chain substituent selected from sulfonic acid, a sulfoneamide group, an aminomethyl group or a phthalimidemethyl group into an organic pigment as a matrix structure. These compounds have a high effect on freedom of aggregation and stability against crystallization. Since, however, the compound which is to be incorporated is derived from a compound having the chemical structure of a pigment, it has inherent intense colouring properties and is therefore extremely limiting when applied to a pigment having a different hue. It is therefore necessary to provide compounds corresponding to individual pigments, which is a significant disadvantage when producing pigment compositions. Further, when a substituents is introduced as a side chan to a pigment for the preparation of the above compounds, it is necessary to dissolve the pigment in concentrated sulfuric acid or fuming sulfuric acid, or to react chlorosulfonic acid with the pigment, in order to introduce a reactive group. Concentrated sulfuric acid and fuming sulfuric acid are dangerous and difficult to handle, limitations are imposed on their production facilities and output. These limitations are disadvantageous for the production of the above compounds.

It is an object of the present invention to provide a pigment composition comprising a colorless or only slightly colored pigment dispersing agent feasible as a pigment dispersing agent for pigments having various hues.

It is another object of the present invention to provide a pigment composition comprising a pigment dispersing agent having a matrix structure other than an organic pigment.

It is further another object of the present invention to provide a pigment composition capable of providing an ink or a coating composition excellent in freedom of aggregation, non-crystallizability and fluidity, and a pigment composition containing a pigment dispersing agent capable of providing the above pigment composition.

According to the present invention, there is provided a pigment composition comprising:
(a) 100 parts by weight of a pigment; and
(b) from 0.1 to 30 parts by weight of a pigment dispersing agent; which pigment dispersing agent is a compound of general formula (I) 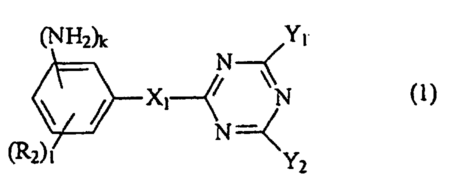
wherein:
- each R₂ is a halogen atom, an amino group, a nitro group, a hydroxyl group, an alkoxy group, a carboxyl group, a sulfonic acid group, a C₁ to C₂₀ alkyl group or a C₁ to C₂₀ alkenyl group, wherein said alkyl or alkenyl group is unsubstituted or substituted by a halogen, a hydroxyl group or a mercapto group;
- k is an integer of from 1 to 3;
- l is an integer of from 0 to 2;
- X is -O- or -NR₃- wherein R₃ is a hydrogen atom, alkyl group or alkenyl group, wherein said alkyl or alkenyl group is unsubstituted or substituted by a halogen, a hydroxyl group or a mercapto group; and
- either Y₁ and Y₂ are the same or different and each represent a group of the general formula (2), (3), (4) or (5) or Y₁ is a group of the general formula (2), (3), (4) or (5) and Y₂ is a hydroxyl group, an alkoxy group or a group of the general formula (6), wherein:
   the general formula (2) is: 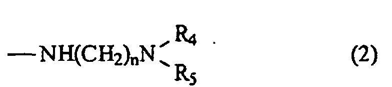
wherein R₄ and R₅ are the same or different and are C₁ to C₂₀ alkyl groups or C₁ to C₂₀ alkenyl groups wherein said alkyl and alkenyl groups are each, individually, unsubstituted or substituted by a halogen, a hydroxyl group or a mercapto group; or R₄ and R₅ form a five- or six-membered heterocyclic ring which may contain one or more further heteroatoms selected from N, O and S, and n is an integer of from 1 to 6;
the general formula (3) is 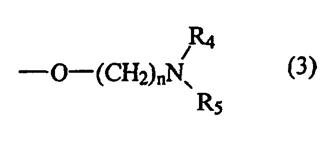 wherein R₄ and R₅ are as defined above;
the general formula (4) is 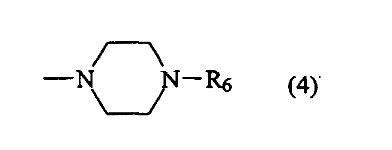 wherein R₆ is an alkyl group or alkenyl group wherein said alkyl or alkenyl group is unsubstituted or substituted by a halogen, a hydroxyl group or a mercapto group;
the general formula (5) is 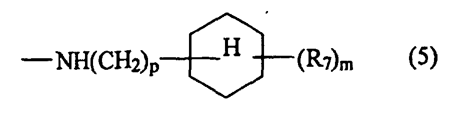 wherein R, represents a group of the general formula (4) as defined above or a group of the general formula (7), p is an integer of from 0 to 6 and m is an integer of 1 or 2; the general formula (6) is 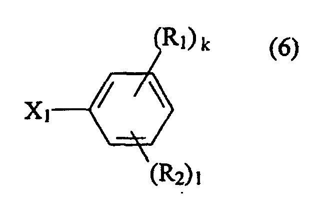 wherein X₁, R₁, R₁, k and I are as defined above;
and the general formula (7) is 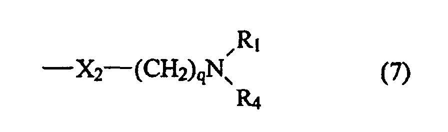 wherein X₂ is a direct bond, -NH- or -O-, q is an integer of from 0 to 6 and R₄ and R₅ are as defined above.

According to the present invention, there is also provided a pigment composition, wherein Y, is a group of the general formula (2).

The present invention further provides a pigment composition as described above, wherein each of R₄ and R₅ is an alkyl group having from 1 to 6 carbon atoms.

Further, the present invention provides a pigment composition as described above, wherein X₁ is -NH-.

The present invention also provides a pigment composition as described above, which is an ink or a coating composition.

Further still, the present invention provides a method for preparing a pigment composition described above, which method comprises mixing, in any order, a pigment, a pigment dispersing agent as described above and an aqueous or non-aqueous vehicle.

The pigment dispersing agent of the pigment composition of the present invention has a triazine ring, an aromatic amino group and an amino group represented by Y₁ in the general formula (1), i.e., Y₁ is a substitutent represented by one of the general formulae (2) to (5).

The alkoxy group of each of R₂ and Y₂ in the general formula (1) is preferably an alkoxy group having from 1 to 20 carbon atoms. Examples thereof include methoxy, ethoxy, n-propoxy, isopropoxy, n-butoxy, isobutoxy, pentoxy and hexoxy.

The substituted or non-substituted alkyl or the substituted or non-substituted alkenyl group included in each of R₂, X₁ and Y₁ are preferably each a substituted or non-substituted alkyl or alkenyl group having 1 to 20 carbon atoms. Examples of the non-substituted alkyl group include methyl, ethyl, n-propyl, isopropyl, n-butyl, isobutyl, pentyl and hexyl. Examples of the non-substituted alkenyl group include vinyl, 1-propenyl, 2-propenyl, isopropenyl, 1-butenyl, 2-butenyl, 2-pentenyl and 2-methylallyl. The substituted alkyl or alkenyl group refers to an alkyl or alkenyl group of which a hydrogen atom is replaced with a halogen such as a chlorine atom or a bromine atom, a hydroxyl group or a mercapto group. Further, a combination of R₄ and R₅ may be a five- or six-membered ring which alkyl groups of R₄ and R₅ form, together with the nitrogen atom to which they are both bonded, and which may further contain a nitrogen atom, an oxygen atom or a sulfur atom. Examples of the above five- or six-membered ring include the following substituents.

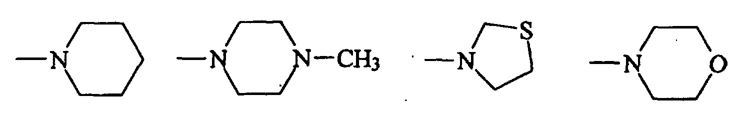

Of the above, Y₁ is preferably a group of the general formula (2), and further, each of R₄ and R₅ is preferably an alkyl group having from 1 to 6 carbon atoms.

The benzene ring of the pigment dispersing agent of the pigment composition of the present invention may have a substituent represented by R₂ in a position where it can be substituted, in addition to the amino group represented by R₁ in the general formula (1). That is, the benzene ring may contain any substitutent selected from a halogen atom, an amino group, a nitro group, a hydroxyl group, an alkoxy group, a carboxyl group, a sulfonic acid group, a substituted or non-substituted alkyl group or a substituted or non-substituted alkenyl group. Further, the benzene ring may have two of the above substituents, and in this case, one substituent may be the same as, or different from, the other.

The pigment dispersing agent of the pigment composition of the present invention has been explained with regard to its structure hereinabove, and examples of the pigment dispersing agent include the following compounds.

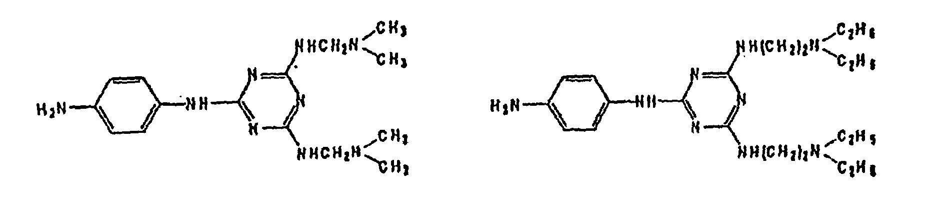

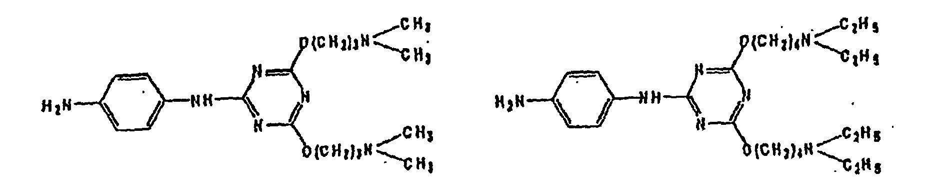

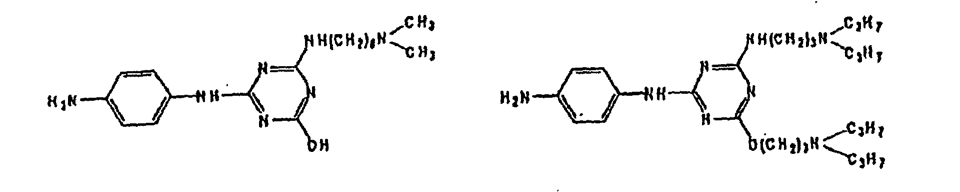

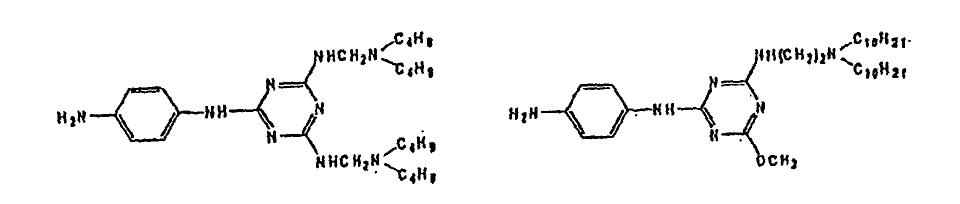

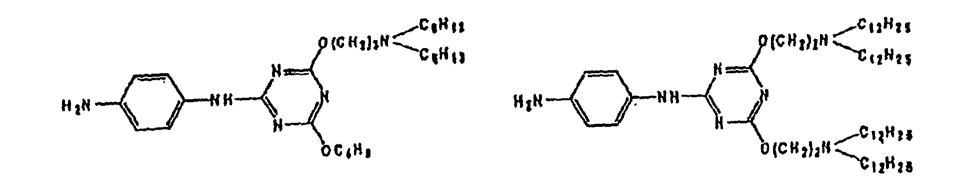

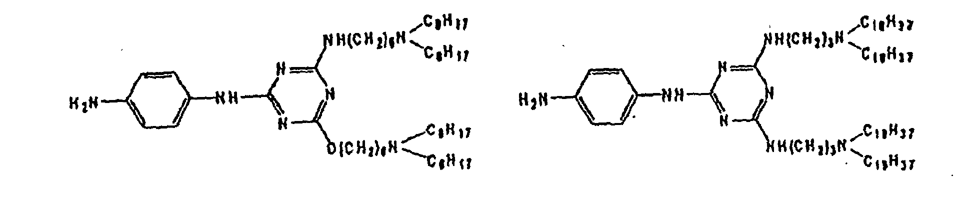

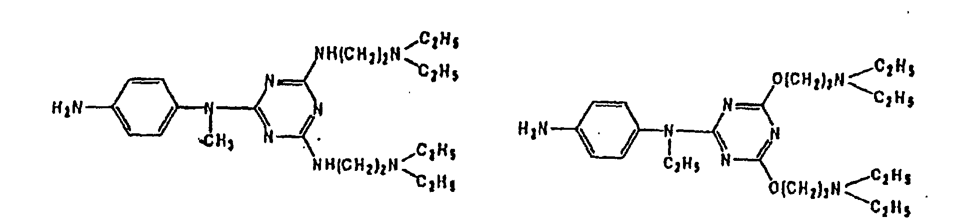

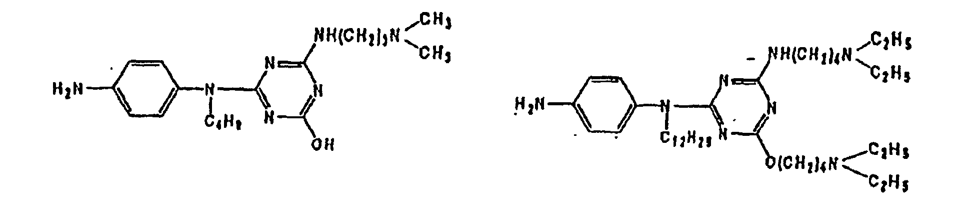

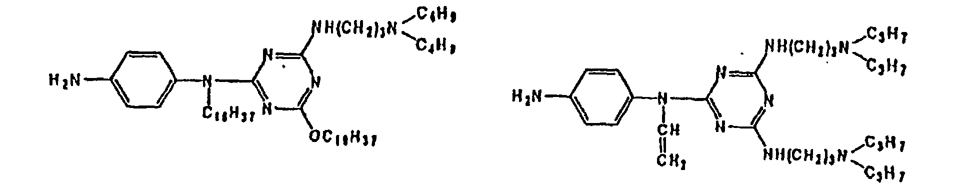

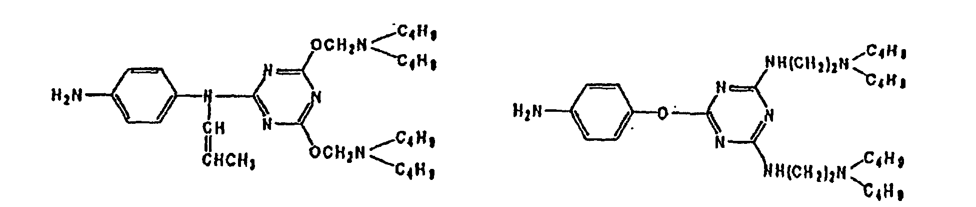

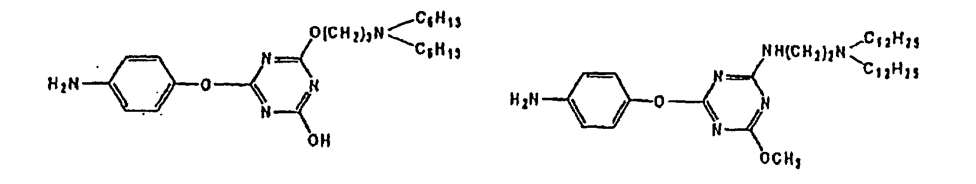

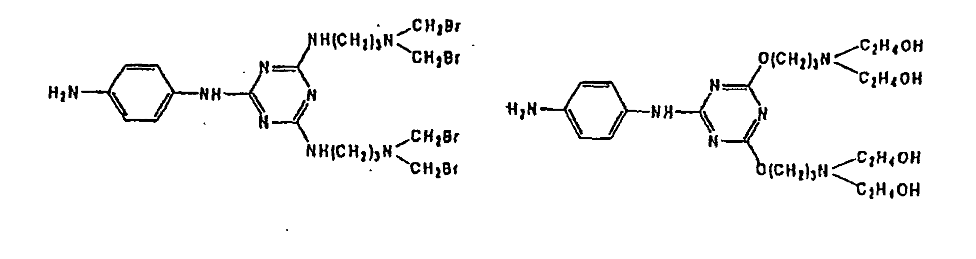

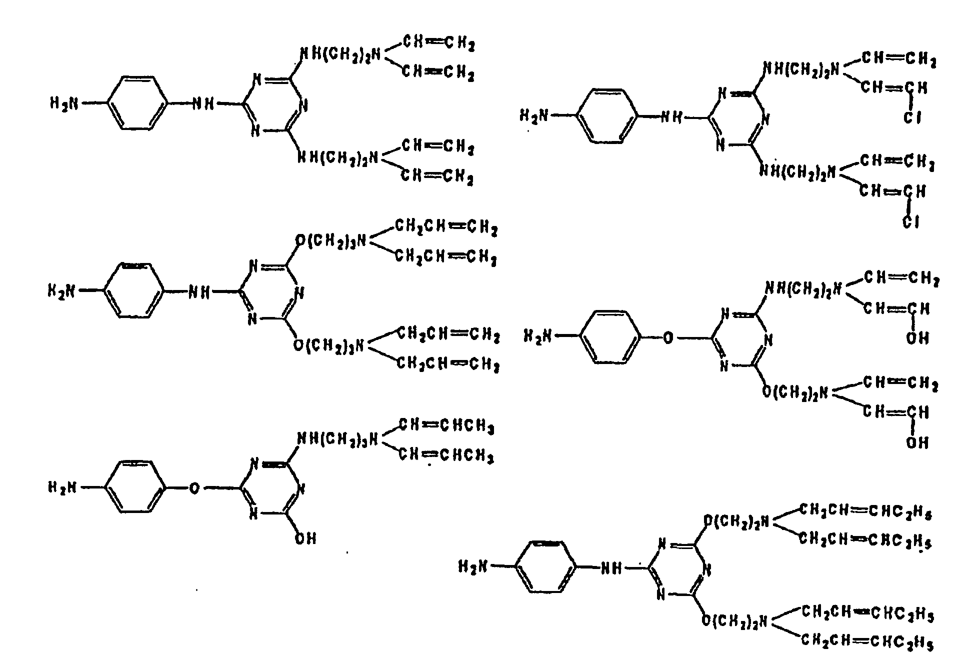

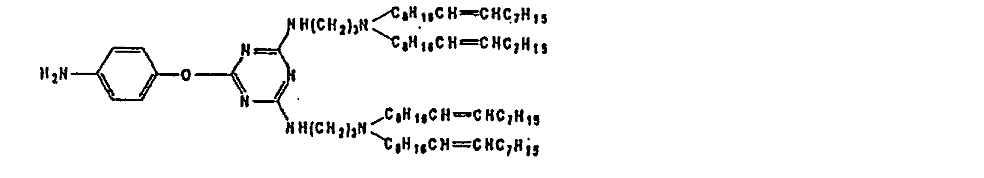

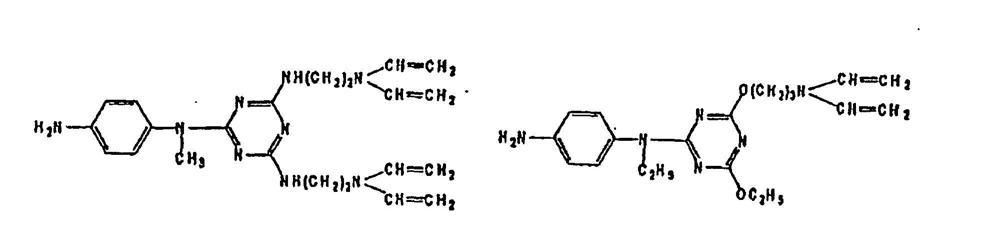

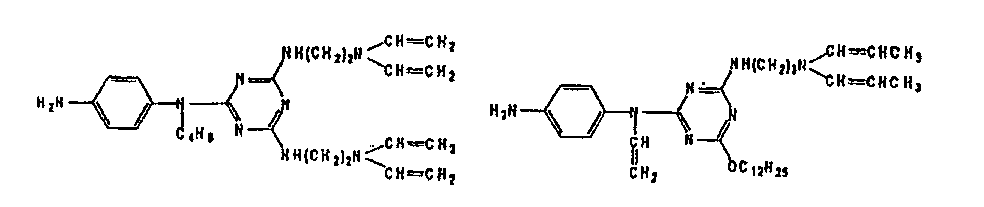

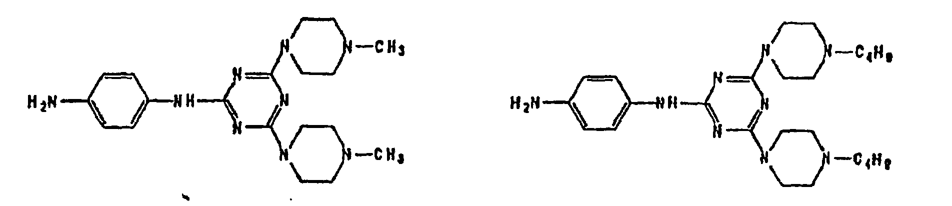

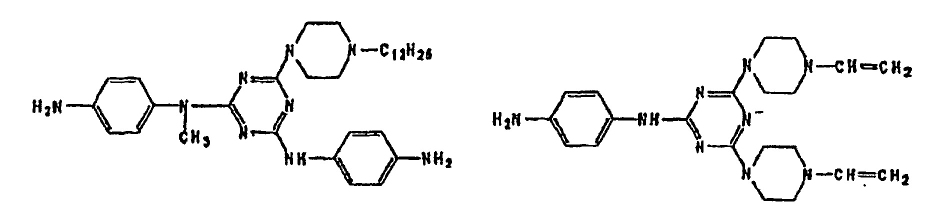

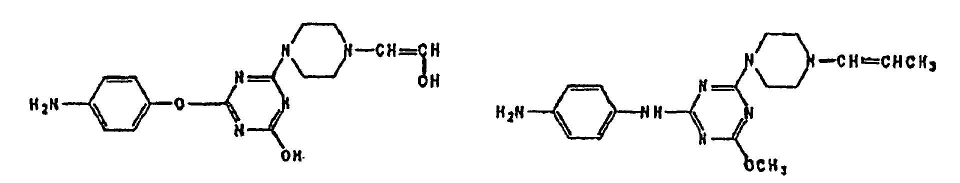

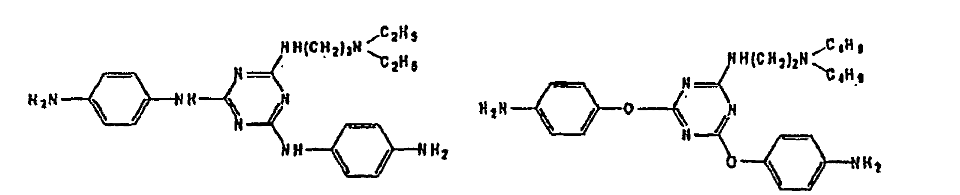

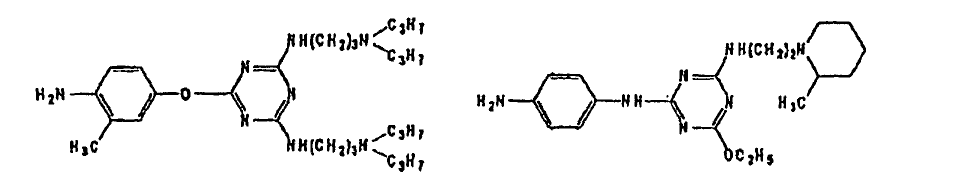

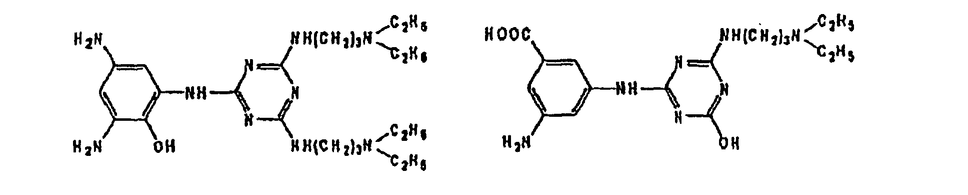

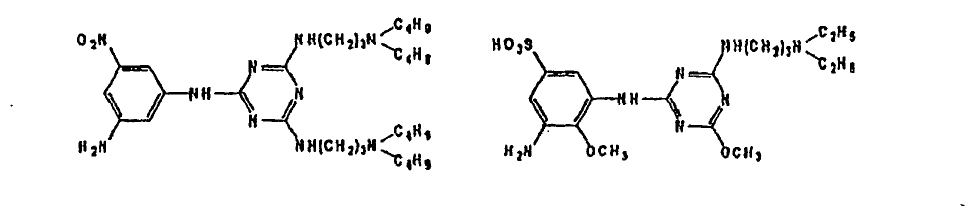

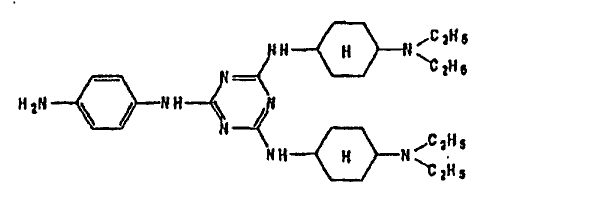

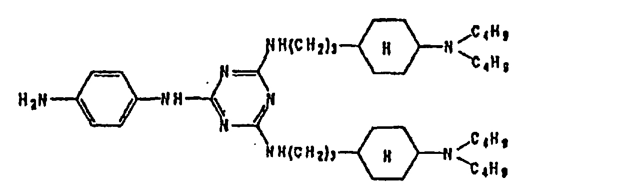

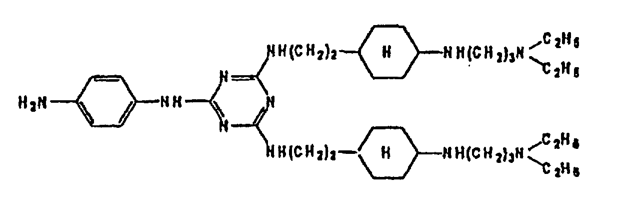

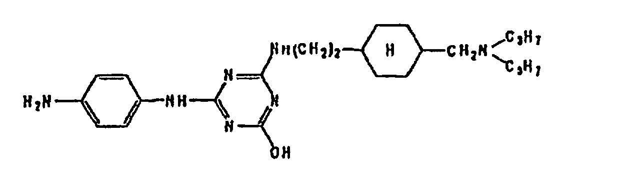

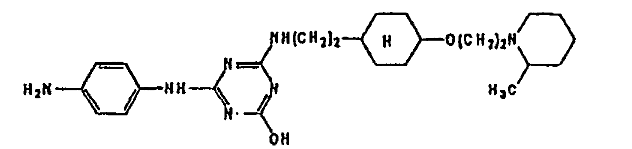

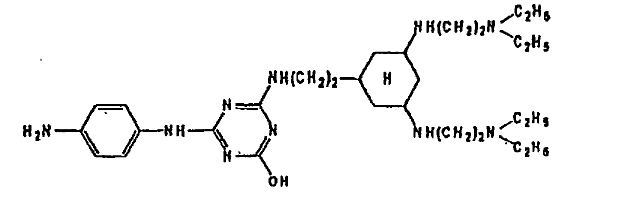

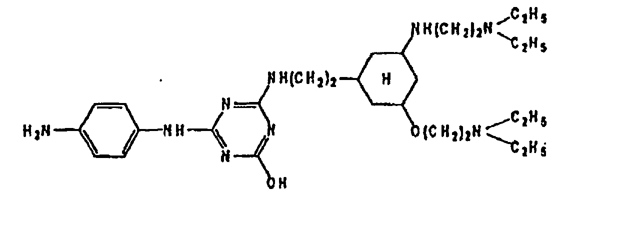

The pigment dispersing agent of the present invention can be produced, for example, by the following method. First, equimolar amounts of p-aminoacetoanilide and cyanuric chloride are allowed to react in water, an acetic acid aqueous solution, an alcohol, a mixture of water with an alcohol or a general organic solvent such as xylol, to obtain a compound (8) of the following formula.

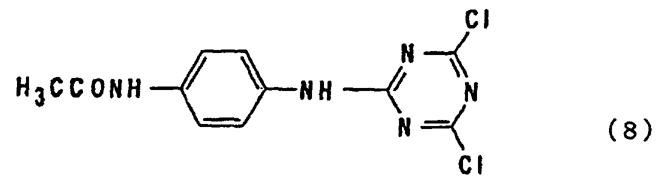

Then, to introduce Y₁ and Y₂, a compound of any one of the general formulae (9) to (12) is reacted with the compound (8) in water or a general organic solvent, to prepare a compound of one of the general formulae (13) to (16).

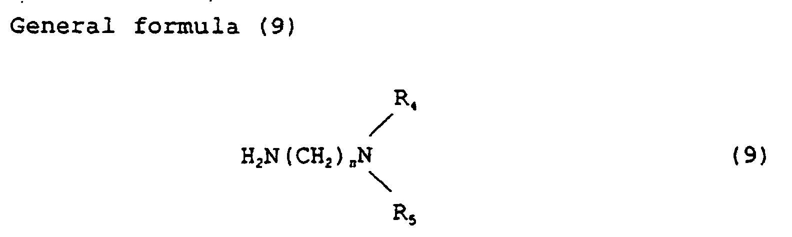

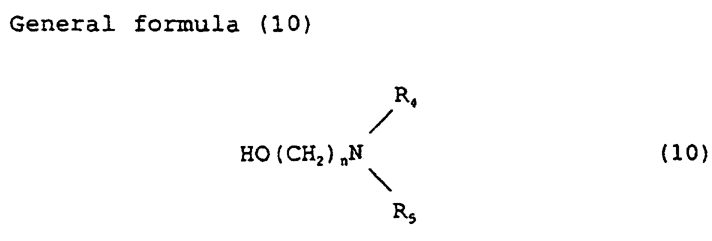

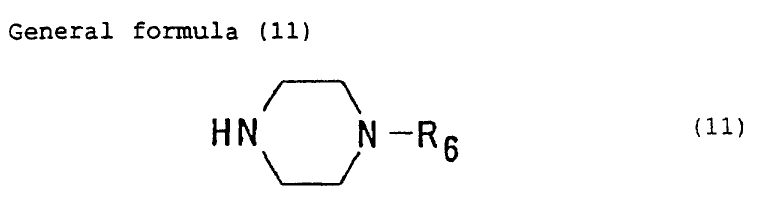

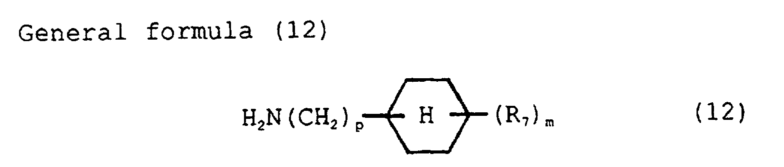

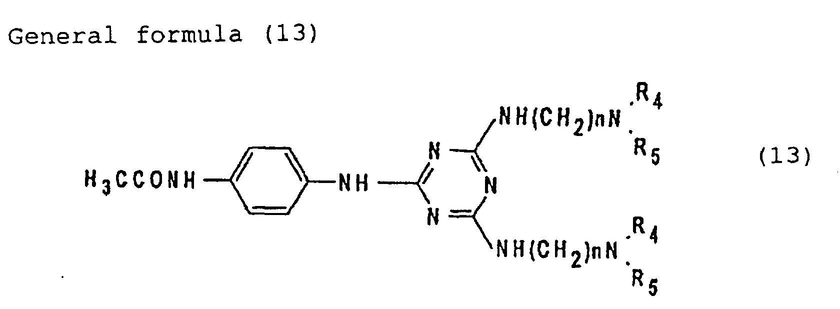

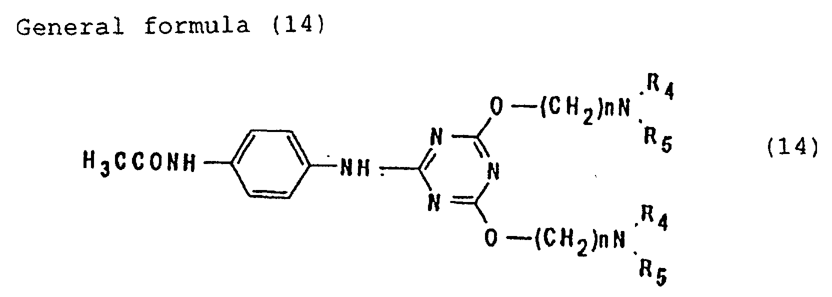

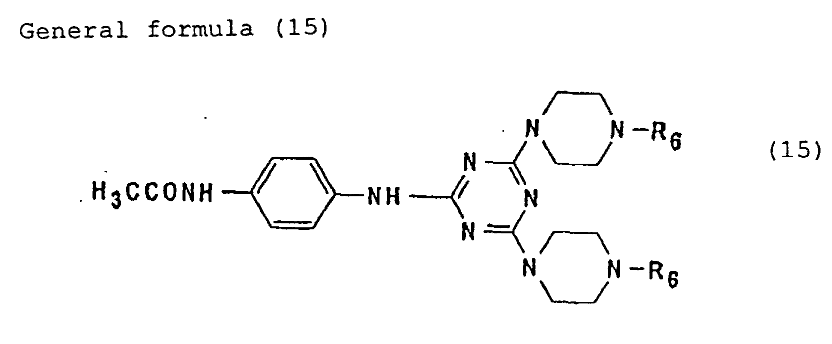

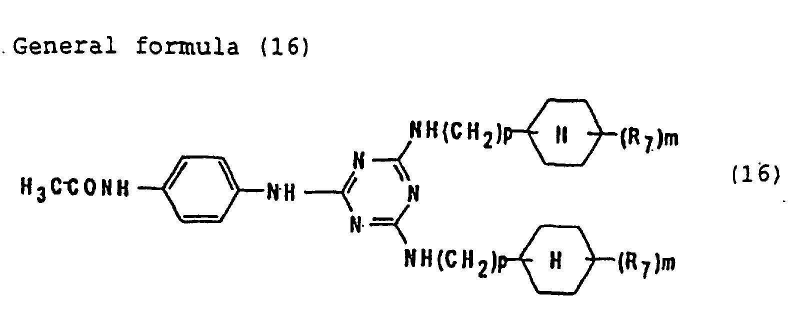

In the above formulae, R₄, R₅, R₆, R₇, m, n and p are as defined in the general formula (1).

Then, the compound of the general formula (13), (14), (15) or (16) is hydrolyzed in the presence of a hydrochloric acid aqueous solution or the like, whereby the pigment dispersing agent of the general formula (1), provided by the pigment composition of the present invention, can be obtained.

In another method, p-nitro-aniline is used in place of p-amino-acetonitrile, to obtain a compound of the general formula (17). The compound of general formula (17) is reacted with a compound of any one of the general formulae (9) to (12), and then the nitro group is reduced, whereby the pigment dispersing agent of general formula (1), of the pigment composition provided by the present invention, can be also obtained.

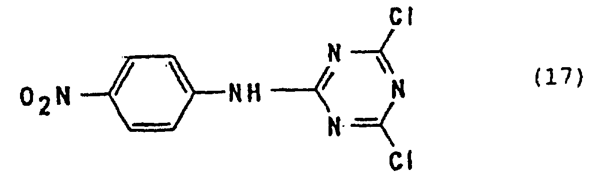

The compound (8) or the compound (17) may be hydrolyzed in the position of one of the chlorine atoms during the reaction to provide a hydroxyl group or an alkoxy group, while the pigment dispersing agent obtained as the end product can be used as a pigment dispersing agent in the pigment composition of the present invention.

Example of the compound of general formula (9) used in the present invention include N,N-dimethylaminoethylamine, N,N-diethylaminoethylamine, N,N-dibutylaminoethylamine, N,N-dimethylaminopropylamine, N,N-diethylaminopropylamine, N,N-dibutylaminopropylamine, N,N-dimethylaminobutylamine, N,N-diethylaminobutylamine, N,N-dipropylaminobutylamine, N,N-dibutylaminobutylamine, N,N-diethylaminohexylamine, N-aminomethylpiperidine, N,N-aminoethylpiperidine, N-aminopropylpiperidine, N-aminoethylpyrrolidine, N-aminopropylpyrrolidone, N-aminomethyl-2-pipecoline, N-aminoethyl-4-pipecoline, N-aminopropyl-2-pipecoline, N-aminoethylmorpholine, N-aminopropylmorpholine, N,N-methyl-laurylaminoptopylamine, N,N-dioleylaminoethylamine and N,N-distearylaminobutylamine.

Examples of the compound of the general formula (10) used in the present invention include N,N-dimethylaminomethanol, N,N-dimethylaminoethanol, N,N-diethylaminoethanol, N,N-dibutylaminomethanol, N,N-dibutylaminoethanol, N,N-dibutylaminopropanol, N,N-diethylaminobutanol, N,N-dioleylbutanol, N-hydroxyethylpiperidine, N-hydroxypropylpiperidine, N-hydroxyethylpipecoline, N-hydroxypropylpipecoline, N-hydroxymethylpyrrolidine, N-hydroxybutylpyrrolidine, N-hydroxyethylmorpholine and N-hydroxybutylmorpholine.

Examples of the compound of the general formula (11) used in the present invention include N-methylpiperazine, N-ethylpiperazine, N-butylpiperazine and 1-cyclopentylpiperazine.

Examples of the compound of the general formula (12) used in the present invention include 1-amino-4-N,N-dimethylaminocyclohexane, 1-amino-4-N,N-diethylaminocyclohexane, 1-amino-4-N,N-dibutyaminocyclohexane, 1-aminomethyl-4-N,N-dimethylaminocyclohexane, 1-aminomethyl-4-N,N-diethylaminocyclohexane, 1-aminomethyl-4-N,N-dibutylaminocyclohexane, 1-aminoethyl-4-N,N-dimethylaminocyclohexane, 1-aminoethyl-4-N,N-diethylaminocyclohexane, 1-aminoethyl-4-N,N-dibutylaminocyclohexane, 1-amino-4-N,N-dimethylaminomethylcyclohexane, 1-amino-4-N,N-dibutylaminomethylcyclohexane, 1-amino-4-N,N-dimethylaminoethylaminocyclohexane, 1-amino-4-N,N-dimethylaminopropylaminocyclohexane, 1-amino-4-N,N-diethylaminoethylaminocyclohexane, 1-amino-3,5-N,N-dimethylaminocyclohexane, 1-amino-3,5-N,N-diethylaminocyclohexane, 1-amino-3,5-N,N-dibutylaminocyclohexane, 1-amino-3,5-N,N-dimethylaminomethylcyclohexane, 1-amino-3,5-N,N-diethylaminomethylcyclohexane, 1-amino-3,5-N,N-dibutylaminomethylcyclohexane, 1-amino-4-N-methylpiperazinecyclohexane, 1-amino-4-N-ethylpiperazinecyclohecane and 1-amino-4-N-butylpiperazinecyclochexane.

The pigment dispersing agent obtained by the above reactions exhibits excellent effects on all generally commercially available pigments. It can be applied, for example, to an organic pigment such as a phthalocyanine pigment, a quinacridone pigment, an isoindolinone pigment, a perylene-perinone pigment, a dioxadine pigment, a diketopyrrolopyrrole pigment, an anthraquinone pigment, a benzoimidazolone pigment and an azo pigment, and an inorganic pigment such as carbon black, titanium oxide, chrome yellow, cadmium yellow, cadmium red, red iron oxide, iron black, flowers of zinc, Prussian blue and ultramarine.

The pigment dispersing agent of the pigment composition provided by present invention has a colorless or a slightly colored hue, so that it is advantageous in that it causes no, or almost no, change in hue when added to a variety of pigments and is excellent in use together with almost all pigments.

The pigment composition of the present invention is a composition comprising a pigment and the pigment dispersing agent of general formula (1).

The amount of the pigment dispersing agent of general formula (1) per 100 parts by weight of the pigment is preferably 0.1 to 30 parts by weight.

When the amount of the pigment dispersing agent of general formula (1) is less than 0.1 part by weight, undesirably, the effect on dispersing the pigment is low. When it exceeds 30 parts by weight, no further affect can be obtained.

The pigment dispersing agent is combined with a pigment to produce the pigment composition of the present invention by the following methods, for example.
1. A pigment and the pigment dispersing agent are mixed to obtain a pigment composition in advance, and the pigment composition is dispersed in a non-aqueous vehicle.
2. A pigment and the pigment dispersing agent are separately added to a non-aqueous vehicle, and then dispersed.
3. A pigment is dispersed in a non-aqueous vehicle, the pigment dispersing agent is dispersed in a separate non-aqueous vehicle, and the resultant dispersions are mixed. In this case, the pigment dispersing agent may be dispersed in a solvent alone.
4. A pigment is dispersed in a non-aqueous vehicle to prepare a pigment dispersion, and the pigment dispersing agent is added to the pigment dispersion.

The intended effect can be obtained by any one of the.above methods.

In the preparation of the pigment composition, the dispersing effect can be fully obtained by simply mixing a pigment powder and the pigment dispersing agent of the present invention, while superior results can be obtained by mechanically mixing a pigment and the pigment dispersing agent with a kneader, a roll, an attritor, a super mill or other pulverizer, by adding a solution containing the pigment dispersing agent of the present invention to a suspension of a pigment in water or an organic solvent to deposit pigment dispersing agent on the surface of each pigment particle, or by co-dissolving an organic pigment and the pigment dispersing agent in a solvent having high dissolving power, such as sulfuric acid, and co-precipitating them in a bad solvent, such as water.

Further, when a pigment or the pigment dispersing agent is dispersed in, or mixed with, a non-aqueous vehicle or a solvent, a dispersing machine such as a dissolver, a high-speed mixer, a homo-mixer, a kneader, a roll mill, a sand mill or an attritor may be used, and in this case, the pigment can be well dispersed.

According to the present invention, therefore, an excellent fluidity such as a decrease in the viscosity of a dispersion and a decrease in structural viscosity, is exhibited as compared with the case using a pigment alone, for example, when used in a non-aqueous vehicle for a gravure ink such as a lime rosin varnish, a polyamide resin varnish or a vinyl chloride resin varnish, an ordinary-drying or baking coating composition such as a nitrocellulose lacquer or an aminoalkyd resin, an acryl lacquer, an aminoacrylic resin baking coating composition or a urethane resin coating composition. At the same time, there are no problems such as color separation or a change in crystal, and a printed product and a coating have excellent gloss so that aesthetically fine products can be obtained.

The pigment dispersing agent described above and the pigment composition of the present invention are not limited to a mixture with a non-aqueous vehicle, and have an excellent dispersing effect when mixed with other printing inks or coating compositions or when used to color plastics.

The present invention will be more specifically explained with reference to Examples hereinafter. In Examples, "part" stands for "part by weight", and "%" stands for "% by weight".

### Preparation Example 1

20 Parts of p-aminoacetoanilide and 25 parts of cyanuric chloride were added to 300 parts of methanol, and the mixture was allowed to react at 20°C or lower for 2 hours. Then, 46 parts of N,N-dibutylaminoethylamine was added, and the mixture was refluxed under heat for 2 hours. Then, 100 parts of hydrochloric acid was added for hydrolysis, methanol was then removed, and 40 parts of sodium hydroxide and 1,000 parts of water were added, followed by filtration, washing with water and drying, to give 64 parts of a pigment dispersing agent (a) having the following structure.

### Pigment dispersing agent (a)

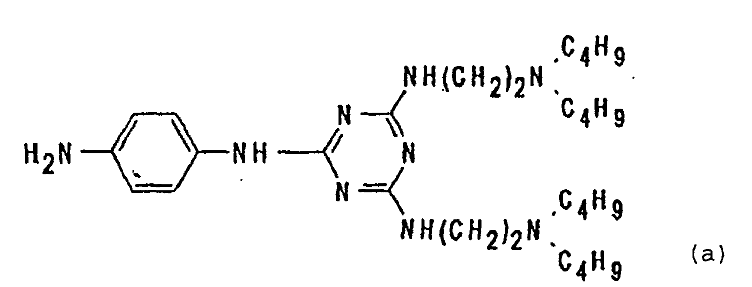

### Preparation Example 2

76 Parts of a pigment dispersing agent (b) having the following structure was obtained in the same manner as in Example 1 except that 46 parts of N,N-dibutylaminoethylamine was replaced with 50 parts of N,N-dibutylaminopropanol.

### Pigment dispersing agent (b)

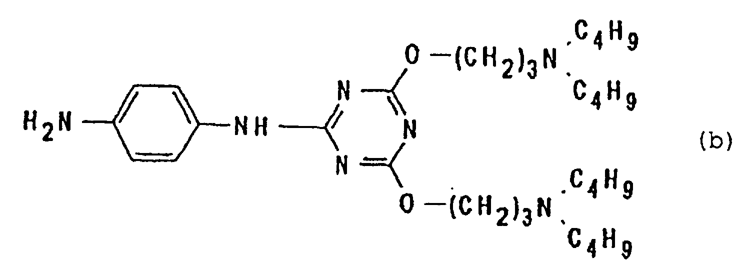

### Preparation Example 3

22 Parts of 3-methyl-4-aminoacetoanilide and 25 parts of cyanuric chloride were added to 300 parts of methanol, and the mixture was allowed to react at 20°C or lower for 2 hours. Then, 12 parts of N,N-dimethylaminoethylamine was added, and the mixture was refluxed under heat for 2 hours. Then, 100 parts of hydrochloric acid was added for hydrolysis, methanol was then removed, and 40 parts of sodium hydroxide and 1,000 parts of water were added, followed by filtration, washing with water and drying, to give 47 parts of a pigment dispersing agent (c) having the following structure.

### Pigment dispersing agent (c)

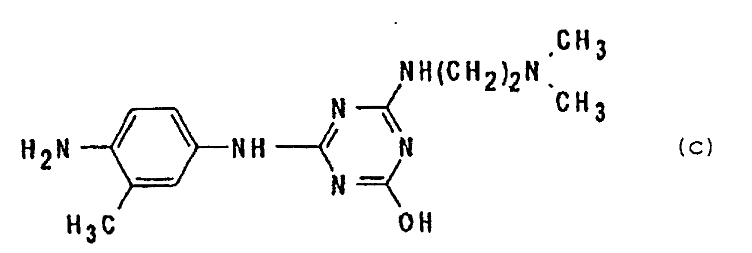

### Preparation Example 4

40 Parts of p-aminoacetoanilide and 25 parts of cyanuric chloride were added to 300 parts of methanol, and the mixture was allowed to react at 30°C for 2 hours. Then, 14 parts of N,N-diethylaminoethylamine was added, and the mixture was refluxed under heat for 2 hours. Then, 100 parts of hydrochloric acid was added for hydrolysis, methanol was then removed, and 40 parts of sodium hydroxide and 1,000 parts of water were added, followed by filtration, washing with water and drying, to give 62 parts of a pigment dispersing agent (d) having the following structure.

### Pigment dispersing agent (d)

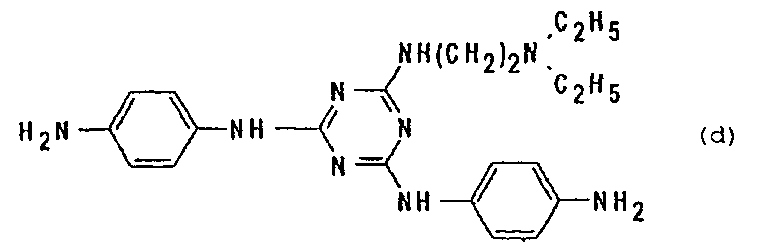

Pigment dispersing agents (e) to (1) having structures shown in Table 1 were synthesized in similar manners.

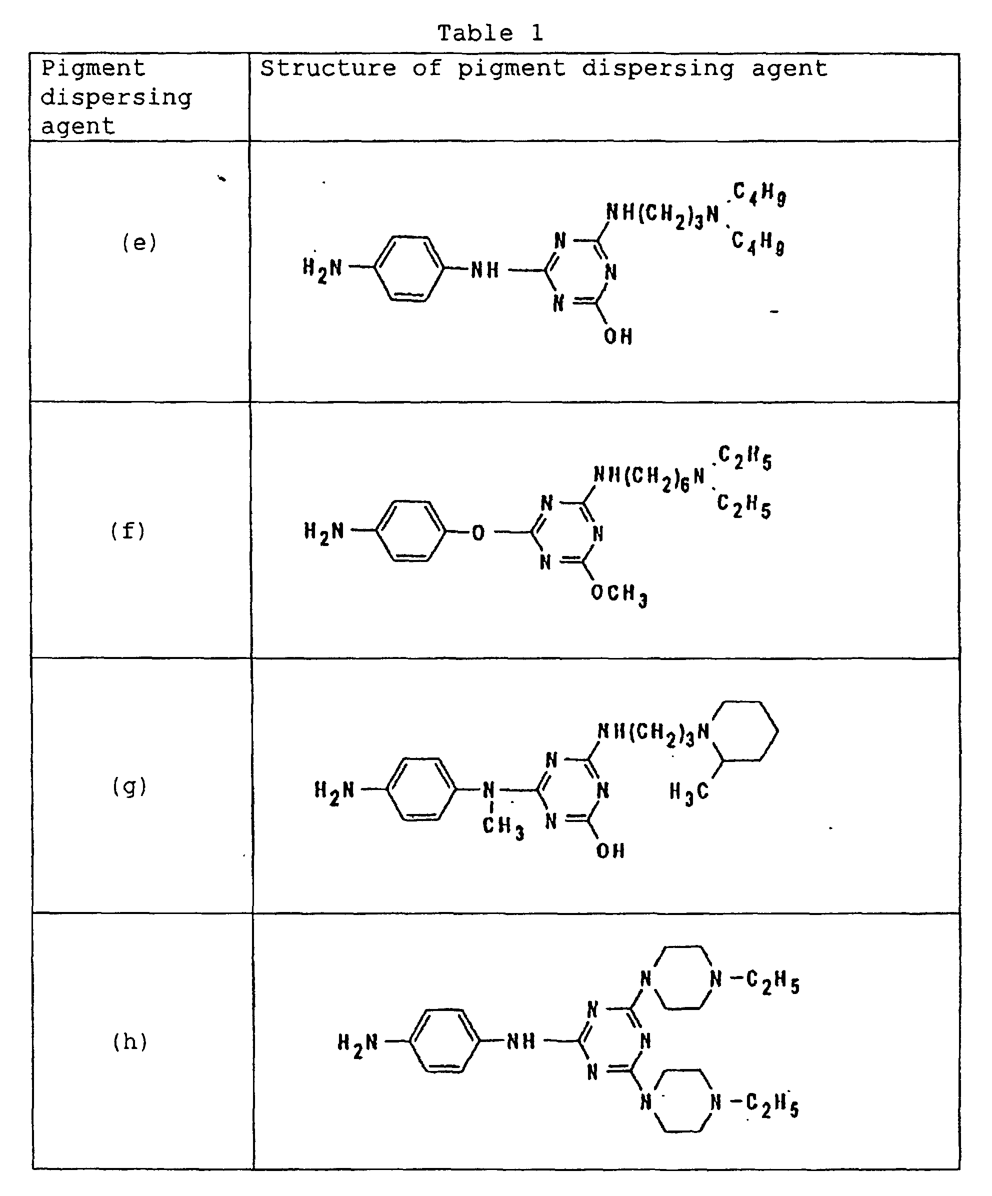

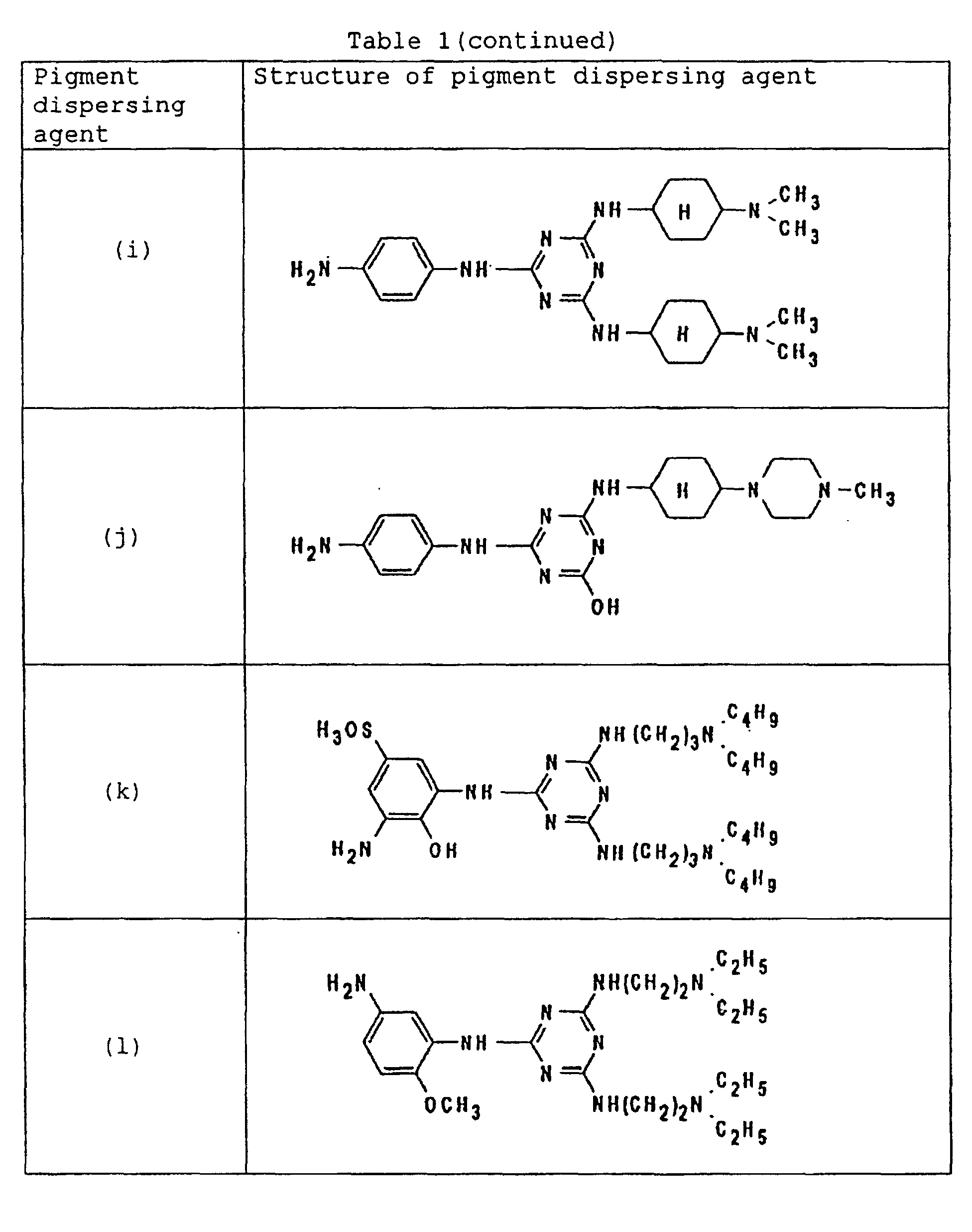

### Examples 1-21 and Comparative Examples 1-15.

Coating compositions were prepared as follows. A pigment alone or a pigment composition containing a pigment and a pigment dispersing agent was dispersed in an aminoalkyd resin varnish for a baking coating composition such that the resultant coating composition had a pigment content of 6 %.

Examples 1 to 21 contain a pigment composition containing a pigment and one of the pigment dispersing agents (a) to (l).

Comparative Examples 1 to 15 contain either a pigment alone or a pigment composition containing a pigment and a pigment dispersing agent (m) or (n) having a pigment structure.

As a pigment dispersing agent having a pigment structure, the following compounds having a copper phthalocyanine structure or a quinacridone structure were used.

Pigment dispersing agent having a copper phthalocyanine structure (m):

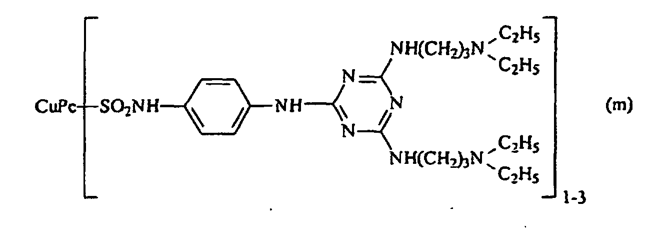

Pigment dispersing agent having a quinacridone structure (n):

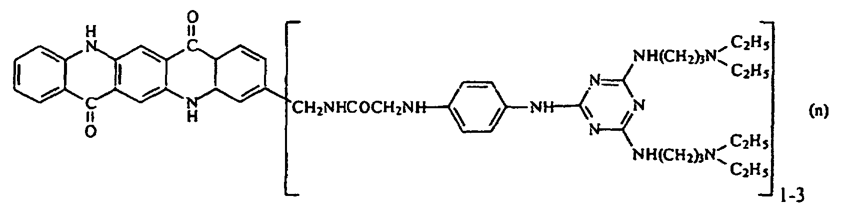

When each of the coating compositions obtained in the above manner was measured for viscosity, the coating compositions containing the pigment dispersing agents of the present invention had low viscosity and showed small thixotropic properties (TI values), and they showed excellent fluidity, as shown in Table 2.

Further, the above coating compositions were viscosity-adjusted to show 20 seconds (25°C) with a ford cup #4, and each of the adjusted coating compositions was air-sprayed to an intermediate-coated plate (steel plate coated with a primer coating composition) so as to form a dry coating having a thickness of about 30 µm. Each plate was allowed to stand for 10 minutes, and then the coatings were baked at 140°C for 30 minutes.

As shown in Table 2, the coating compositions containing the pigment dispersing agents of the present invention showed excellent gloss over compositions in Comparative Examples. Further, the coating compositions containing the pigment dispersing agents of the present invention did not foul the hues of matrix pigments and showed hues equivalent to the hues of the pigments alone.

With regard to freedom of aggregation and non-crystallizability, which is a problem in practical use, the coating compositions were evaluated for stability against color separation as follows.

A coating composition was diluted with a base coating composition (prepared by dispersing titanium oxide in an aminoalkyd resin varnish) so as to have a pigment/titanium oxide ratio of 1/10, whereby a tinting shade coating composition was prepared. The tinting shade coating composition was viscosity-adjusted with xylene to show 20 seconds (25°C) with a ford cup, and the adjusted coating composition was placed in a test tube. When the glass wall of the test tube was observed for a change, the coating compositions containing the pigment dispersing agents of the present invention showed a small color separation with the passage of time as compared with the coating compositions in the Comparative Examples.

**Table 2**

| Ex. CEx. | Pigment | Pigment dispersing agent | Amount ratio of pigment /pigment dispersing agent | Fluidity (cps) | | | Gloss % | Color difference |
|---|---|---|---|---|---|---|---|---|
| | | | | 6 rpm | 60 rpm | TI value | 20°G | ΔE |
| CEx.1 | C.I. Pigment Blue 15:1 (Phthalocyanine pigment) | - | 100/0 | 7.88 (7,880) | 2.16 (2,160) | 3.65 | 65.5 | STD |
| CEx.2 | | m | 95/5 | 0.58 (580) | 0.53 (530) | 1.09 | 82.0 | 0.87 |
| Ex.1 | | a | 95/5 | 0.55 (550) | 0.5 (500) | 1.10 | 82.5 | 0.23 |
| Ex.2 | | b | 95/5 | 0.53 (530) | 0.48 (480) | 1.10 | 84.0 | 0.19 |
| CEx.3 | C.I. Pigment Green 36 (Phthalocyanine pigment) | - | 100/0 | 3.5 (3,500) | 1.02 (1,020) | 3.43 | 69.8 | STD |
| CEx.4 | | m | 95/5 | 0.4 (400) | 0.355 (355) | 1.13 | 80.1 | 1.65 |
| Ex.3 | | e | 95/5 | 0.3 (300) | 0.28 (280) | 1.07 | 84.3 | 0.20 |
| Ex.4 | | f | 95/5 | 0.38 (380) | 0.365 (365) | 1.04 | 83.0 | 0.25 |
| CEx.5 | C.I. Pigment Red 122 (Quinacridone pigment) | - | 100/0 | 5.68 (5,680) | 1.150 (1,150) | 4.94 | 65.0 | STD |
| CEx.6 | | n | 95/5 | 0.4 (400) | 0.38 (380) | 1.05 | 84.0 | 0.58 |
| Ex.5 | | c | 95/5 | 0.31 (310) | 0.31 (310) | 1.00 | 84.3 | 0.12 |
| Ex.6 | | l | 90/10 | 0.54 (540) | 0.52 (520) | 1.04 | 83.7 | 0.18 |
| CEx.7 | C.I. Pigment Red 254 (Diketopyrropyrrol pigment) | - | 100/0 | 2.92 (2,920) | 1.19 (1,190) | 2.45 | 66.2 | STD |
| CEx.8 | | n | 95/5 | 0.54 (540) | 0.5 (500) | 1.40 | 81.3 | 1.43 |
| Ex.7 | | h | 95/5 | 0.44 (440) | 0.42 (420) | 1.05 | 83.1 | 0.28 |
| Ex.8 | | k | 90/10 | 0.41 (410) | 0.41 (410) | 1.00 | 84.0 | 0.24 |
| CEx.9 | C.I. Pigment Red 177 (Anthraquinone pigment) | - | 100/0 | 3.35 (3,350) | 1.59 (1,590) | 2.11 | 64.2 | STD |
| CEx.10 | | n | 95/5 | 0.4 (400) | 0.38 (380) | 1.05 | 83.2 | 1.18 |
| Ex.9 | | g | 95/5 | 0.3 (300) | 0.3 (300) | 1.00 | 86.0 | 0.25 |
| Ex.10 | | j | 90/10 | 0.33 (330) | 0.33 (330) | 1.00 | 84.8 | 0.30 |
| CEx.11 | C.I. Pigment Red 178 (Perylene pigment) | - | 100/0 | (8.95) 8,950 | 1.89 (1,890) | 4.73 | 69.2 | STD |
| Ex.11 | | a | 95/5 | 0.4 (400) | 0.4 (400) | 1.00 | 84.0 | 0.18 |
| Ex.12 | | d | 90/10 | 0.64 (640) | 0.6 (600) | 1.07 | 81.5 | 0.24 |
| Ex.13 | | i | 95/5 | 0.44 (440) | 0.44 (440) | 1.00 | 82.2 | 0.22 |
| CEx.12 | C.I. Pigment Violet 23 (Dioxadine pigment) | - | 100/0 | 3.52 (3,520) | 1.35 (1,350) | 2.61 | 68.2 | STD |
| Ex.14 | | f | 90/10 | 0.61 (610) | 0.58 (580) | 1.05 | 82.0 | 0.10 |
| Ex.15 | | h | 90/10 | 0.55 (550) | 0.55 (550) | 1.00 | 83.5 | 0.15 |
| CEx.13 | C.I. Pigment Yellow 154 (Benzimidazolone pigment) | - | 100/0 | 2.92 (2,920) | 1.02 (1,020) | 2.86 | 70.2 | STD |
| Ex.16 | | a | 90/10 | 0.42 (420) | 0.4 (400) | 1.05 | 84.0 | 0.27 |
| Ex.17 | | i | 90/10 | 0.4 (400) | 0.4 (400) | 1.00 | 84.2 | 0.31 |
| CEx.14 | C.I. Pigment Yellow 14 (Disazo pigment) | - | 100/0 | 11.4 (11,400) | 4.2 (4,200) | 2.71 | 65.2 | STD |
| Ex. 18 | | a | 90/10 | 3.42 (3,420) | 1.84 (1,840) | 1.86 | 81.0 | 0.21 |
| Ex. 19 | | e | 90/10 | 3.84 (3,840) | 2.06 (2,060) | 1.86 | 80.2 | 0.25 |
| CEx. 15 | C.I. Pigment Black 7 (Carbon pigment) | - | 100/0 | 9.58 (9,580) | 3.43 (3,430) | 2.79 | 61.8 | STD |
| Ex. 20 | | e | 95/5 | 0.72 (720) | 0.65 (650) | 1.11 | 83.0 | 0.08 |
| Ex. 21 | | j | 95/5 | 0.8 (800) | 0.75 (750) | 1.07 | 81.2 | 0.11 |
| Ex. = Example, CEx. = Comparative Example Fluidity: Measured with Brookfield viscometer at 20°C. TI value = Viscosity at 6 rpm/viscosity at 60 rpm Colour difference: Difference from product of a pigment alone (ΔE value) | | | | | | | | |

The pigment compositions containing the above pigment dispersing agents (a) to (l) caused no aggregation when used in nitrocellulose lacquer acrylic resin coating compositions and gravure inks, and they thus showed excellent fluidity.

As explained above, not only the pigment composition is excellent in freedom of aggregation, non-crystallizability and fluidity, but also an ink and a coating composition obtained therefrom are excellent in gloss and hue.

## Claims

1. A pigment composition comprising: (a) 100 parts by weight of a pigment; and (b) from 0.1 to 30 parts by weight of a pigment dispersing agent; which pigment dispersing agent is a compound of general formula (I), 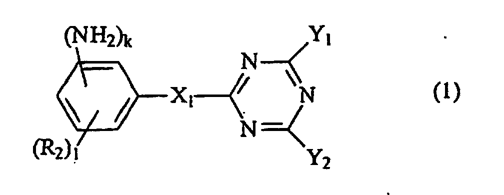 wherein:
each R₂,which may be the same or different is a halogen atom, an amino group, a nitro group, a hydroxyl group, an alkoxy group, a carboxyl group, a sulfonic acid group, a C₁ to C₂₀ alkyl group or a C₁ to C₂₀ alkenyl group, wherein said alkyl or alkenyl group is unsubstituted or substituted by a halogen, a hydroxyl group or a mercapto group;
- k is an integer of from 1 to 3;
- l is an integer of from 0 to 2;
- X₁is -O- or -NR₃- wherein R₃ is a hydrogen atom, alkyl group or an alkenyl group, wherein said alkyl or alkenyl group is unsubstituted or substituted by a halogen, a hydroxyl group or a mercapto group; and
- either Y₁ and Y₂ are the same or different and each represent a group of the general formula (2), (3), (4) or (5) or Y₁ is a group of the general formula (2), (3), (4) or (5) and Y₂ is a hydroxyl group, an alkoxy group or a group of the general formula (6) wherein the general formula (2) is 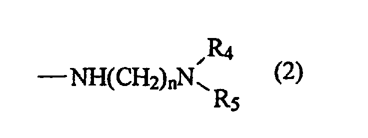
wherein R₄ and R₅ are the same or different and are C₁ to C₂₀ alkyl groups or C₁ to C₂₀ alkenyl groups, wherein said alkyl and alkenyl groups are each, individually, unsubstituted or substituted by a halogen, a hydroxyl group or a mercapto group; or R₄ and R₅, together with the N atom to which they are attached, form a five- or six-membered heterocyclic ring which may contain one or more further heteroatoms selected from N, 0 and S, and n is an integer of from 1 to 6;
the general formula (3) is 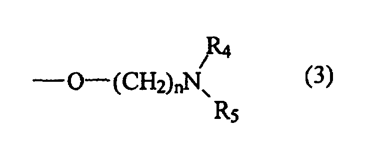 wherein R₄ and R₅ are as defined above;
the general formula (4) is 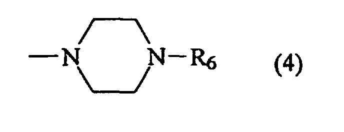 wherein R₆ is an alkyl group or an alkenyl group wherein said alkyl or alkenyl group is unsubstituted or substituted by a halogen, a hydroxyl group or a mercapto group;
the general formula (5) is 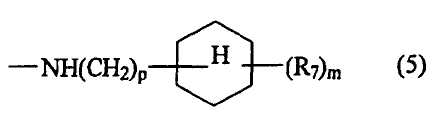 wherein R₇ represents a group of the general formula (4) as defined above, or a group of the general formula (7), p is an integer of from 0 to 6 and m is an integer of 1 to 2;
the general formula (6) is 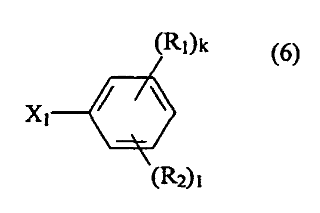 wherein X₁, R₁, R₂, k and l are as defined above; and the general formula (7) is 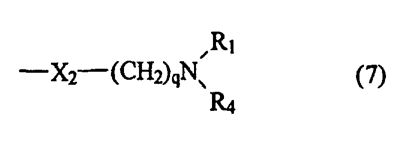 wherein X₂ is a direct bond, -NH- or -O-, q is an integer of from 0 to 6 and R₄ and R₅ are as defined above.

2. A pigment composition according to claim 1, wherein Y₁ is a group of the general formula (2).

3. A pigment composition according to claim 1 or 2, wherein each of R₄ and R₅ is an alkyl group having from 1 to 6 carbon atoms.

4. A pigment composition according to any one of the preceding claims, wherein X₁ is -NH-.

5. A pigment composition according to any one of claims 1 to 4 which is an ink or a coating composition.

6. A method for preparing a pigment composition according to any one of claims 1 to 5 which method comprises mixing, in any order, a pigment, a pigment dispersing agent as defined in any one of claims 1 to 5, and an aqueous or non-aqueous vehicle.

## Patentansprüche

1. Pigment-Zusammensetzung, welche umfasst: (a) 100 Gewichtsanteile eines Pigments; und (b) 0,1 bis 30 Gewichtsanteile eines Pigment-Dispersionsmittels; welches Pigment-Dispersionsmittel eine Verbindung ist der allgemeinen Formel (I), 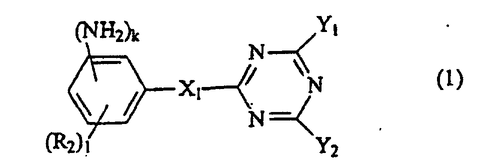 worin:
jedes R₂ das gleich oder verschieden sein kann, ein Halogenatom, eine Aminogruppe, eine Nitrogruppe, eine Hydroxylgruppe, eine Alkoxygruppe, eine Carboxylgruppe, eine Sulfonsäuregruppe, eine C₁ bis C₂₀-Alkylgruppe oder eine C₁ bis C₂₀-Alkenylgruppe ist, wobei die Alkyl- oder Alkenylgruppe unsubstituiert oder durch ein Halogen, eine Hydroxylgruppe oder eine Mercaptogruppe substituiert ist;
- k eine ganze Zahl von 1 bis 3 ist;
- l eine ganze Zahl von 0 bis 2 ist;
- X₁ -O- oder -NR₃- ist, worin R₃ ein Wasserstoffatom, eine Alkylgruppe oder eine Alkenylgruppe ist, wobei die Alkyl- oder Alkenylgruppe unsubstituiert oder durch ein Halogen, eine Hydroxylgruppe oder eine Mercaptogruppe substituiert ist, und
- Y₁ und Y₂ gleich oder verschieden sind und jedes für eine Gruppe der allgemeinen Formel (2), (3), (4) oder (5) steht oder Y₁ eine Gruppe der allgemeinen Formel (2), (3), (4) oder (5) ist und Y₂ eine Hydroxylgruppe, eine Alkoxygruppe oder eine Gruppe der allgemeinen Formel (6) ist, wobei die allgemeine Formel (2) die folgende ist: 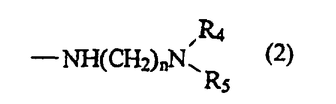
worin R₄ und R₅ gleich oder verschieden sind und C₁ bis C₂₀-Alkylgruppen oder C₁ bis C₂₀-Alkenylgruppen sind, wobei die Alkyl- und Alkenylgruppen jeweils einzeln unsubstituiert oder durch ein Halogen, eine Hydroxylgruppe oder eine Mercaptogruppe substituiert sind; oder R₄ und R₅, zusammen mit dem N-Atom, an das sie gebunden sind, einen fünf- oder sechsgliedrigen heterocyclischen Ring bilden, welcher ein oder mehrere weitere Heteroatome enthalten kann, gewählt aus N, O und S, und n eine ganze Zahl von 1 bis 6 ist;
die allgemeine Formel (3) die folgende ist: 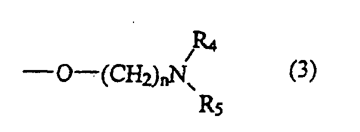 worin R₄ und R₅ wie oben definiert sind;
die allgemeine Formel (4) die folgende ist: 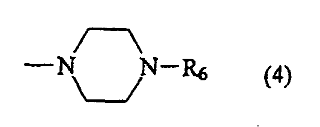 worin R₆ eine Alkylgruppe oder eine Alkenylgruppe ist, wobei die Alkyl- und Alkenylgruppe unsubstituiert oder durch ein Halogen, eine Hydroxylgruppe oder eine Mercaptogruppe substituiert ist;
die allgemeine Formel (5) die folgende ist: 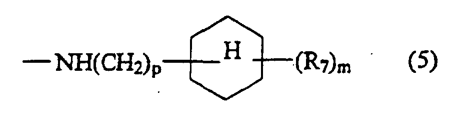 worin R₇ für eine Gruppe der allgemeinen Formel (4) steht, wie oben definiert, oder eine Gruppe der allgemeinen Formel (7),
p eine ganze Zahl von 0 bis 6 ist und m eine ganze Zahl von 1 bis 2 ist;
die allgemeine Formel (6) die folgende ist: 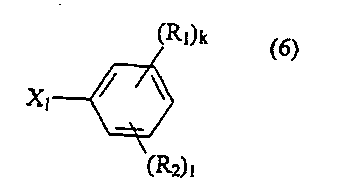 worin X₁, R₁, R₂, k und l wie oben definiert sind; und
die allgemeine Formel (7) die folgende ist: 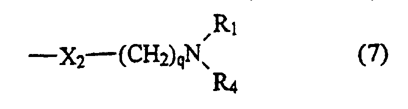 worin X₂ eine direkte Bindung, -NH- oder -O- ist, q eine ganze Zahl von 0 bis 6 ist und R₄ und R₅ wie oben definiert sind.

2. Pigment-Zusammensetzung nach Anspruch 1, worin Y₁ eine Gruppe der allgemeinen Formel (2) ist.

3. Pigment-Zusammensetzung nach Anspruch 1 oder 2, worin jedes von R₄ und R₅ eine Alkylgruppe mit 1 bis 6 Kohlenstoffatomen ist.

4. Pigment-Zusammensetzung nach einem der vorangehenden Ansprüche, worin X₁ -NH- ist.

5. Pigment-Zusammensetzung nach einem der Ansprüche 1 bis 4, welche eine Tinte oder eine Beschichtungszusammensetzung ist.

6. Verfahren zur Herstellung einer Pigment-Zusammensetzung nach einem der Ansprüche 1 bis 5, welches Verfahren das Mischen, in beliebiger Reihenfolge, eines Pigments, eines Pigment-Dispersionsmittels, wie in einem der Ansprüche 1 bis 5 definiert, und eines wässrigen oder nicht-wässrigen Bindemittels umfasst.

## Revendications

1. Composition pigmentaire comprenant : (a) 100 parties en poids d'un pigment ; et (b) de 0,1 à 30 parties en poids d'un agent de dispersion de pigment ; lequel agent de dispersion de pigment est un composé ayant la formule générale (I), 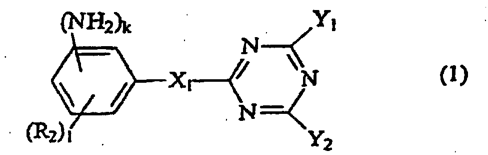 où :
chacun des R₂, qui peuvent être identiques ou différents, est un atome d'halogène, un groupe amino, un groupe nitro, un groupe hydroxyle, un groupe alkoxy, un groupe carboxyle, un groupe acide sulfonique, un groupe alkyle en C₁ à C₂₀ ou un groupe alkényle en C₁ à C₂₀, ledit groupe alkyle ou ledit groupe alkényle étant non substitué ou substitué par un halogène, un groupe hydroxyle ou un groupe mercapto ;
- k est un nombre entier de 1 à 3 ;
- l est un nombre entier de 0 à 2 ;
- X₁ est -O- ou -NR3- où R₃ est un atome d'hydrogène, un groupe alkyle ou un groupe alkényle, ledit groupe alkyle ou ledit groupe alkényle étant non substitué ou substitué par un halogène, un groupe hydroxyle ou un groupe mercapto ; et
- Y₁ et Y₂ sont identiques ou différents et chacun représente un groupe ayant la formule générale (2), (3), (4) ou (5) ou Y₁ est un groupe ayant la formule générale (2), (3), (4) ou (5) et Y₂ est un groupe hydroxyle, un groupe alkoxy ou un groupe ayant la formule générale (6), la formule générale (2) étant la suivante 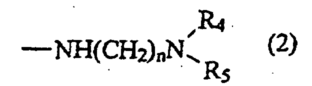
où R₄ et R₅ sont identiques ou différents et sont des groupes alkyle en C₁ à C₂₀ ou des groupes alkényle en C₁ à C₂₀, lesdits groupes alkyle et alkényle étant chacun, individuellement, non substitué ou substitué par un halogène, un groupe hydroxyle ou un groupe mercapto ; ou R₄ et R₅, ensemble avec l'atome N auquel ils sont attachés, forment un anneau hétérocyclique à cinq ou six chaînons qui peut contenir un ou plusieurs autres hétéroatomes sélectionnés parmi N, O et S, et n est un nombre entier de 1 à 6 ;
la formule générale (3) est la suivante 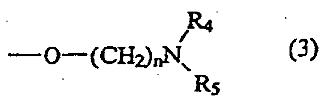 où R₄ et R₅ sont comme défini ci-dessus ;
la formule générale (4) est la suivante 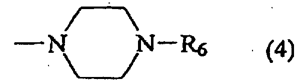 où R₆ est un groupe alkyle ou un groupe alkényle, ledit groupe alkyle ou ledit groupe alkényle étant non substitué ou substitué par un halogène, un groupe hydroxyle ou un groupe mercapto ;
la formule générale (5) est la suivante 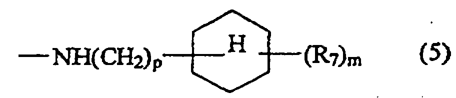 où R₇ représente un groupe ayant la formule générale (4) comme défini ci-dessus, ou un groupe ayant la formule générale (7), p est un nombre entier compris de 0 à 6 et m est un nombre entier de 1 à 2 ;
la formule générale (6) est la suivante 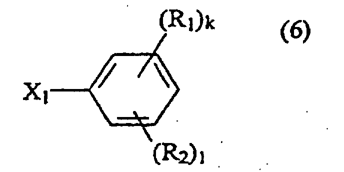 où X₁, R₁, R₂, k et l sont comme défini ci-dessus, et
la formule générale (7) est la suivante 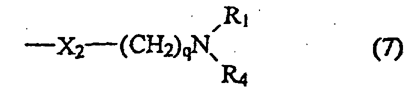 où X₂ est une liaison directe, -NH- ou -O-, q est un nombre entier de 0 à 6 et R₄ et R₅ sont comme défini ci-dessus.

2. Composition pigmentaire selon la revendication 1, dans laquelle Y₁ est un groupe ayant la formule générale (2).

3. Composition pigmentaire selon la revendication 1 ou 2, dans laquelle R₄ et R₅ sont chacun un groupe alkyle ayant de 1 à 6 atomes de carbone.

4. Composition pigmentaire selon l'une quelconque des revendications précédentes, dans laquelle X₁ est -NH-.

5. Composition pigmentaire selon l'une quelconque des revendications 1 à 4, qui est une encre ou une composition de revêtement.

6. Procédé pour préparer une composition pigmentaire selon l'une quelconque des revendications 1 à 5, lequel procédé comporte le mélange, dans un ordre quelconque, d'un pigment, d'un agent de dispersion de pigment comme défini dans l'une quelconque des revendications 1 à 5, et d'un véhicule aqueux ou non-aqueux.
